# EUROPÄISCHE PATENTSCHRIFT

(11) **EP 0 858 773 B1**
(45) Veröffentlichungstag und Bekanntmachung des Hinweises auf die Patenterteilung: **11.09.2002**
(21) Anmeldenummer: 98102209.8
(22) Anmeldetag: 09.02.1998
(51) Int. Cl.: A61B 6/14, A61B 6/06

(54) **Vorrichtung zur Erstellung von Röntgenaufnahmen von Körperteilen eines Menschen**
Device for producing X-rays of parts of a human body
Appareil pour produire des radiographies des parties du corps humain

(30) Priorität: 17.02.1997 DE 19706102
(43) Veröffentlichungstag der Anmeldung: 19.08.1998
(60) Teilanmeldung: 02006254.3
(73) Patentinhaber: Sirona Dental Systems GmbH, 64625 Bensheim (DE)
(72) Erfinder: Zeller, Uwe, Dipl.-Ing. (FH), 61267 Neu-Anspach (DE); Günther, Werner, Ing.grad., 64625 Bensheim (DE); Schulze-Ganzlin, Ulrich, Dipl.-Ing., 64653 Lorsch (DE); Döbert, Michael, 64653 Lorsch (DE)
(74) Vertreter: Sommer, Peter

(56) Entgegenhaltungen:
- EP-A- 0 141 448
- EP-A- 0 632 994
- DE-U- 9 103 670
- US-A- 4 188 537
- US-A- 5 600 699

## Beschreibung

Die Erfindung bezieht sich auf eine Vorrichtung zur Erstellung von Röntgenaufnahmen von Körperteilen eines Menschen, insbesondere von Röntgen-Schichtaufnahmen vom Kiefer oder Schädel eines Patienten, bei dem ein von einer Strahlenquelle erzeugtes und durch eine Blendenöffnung einer Primärblende begrenztes Strahlenbündel nach Durchdringung des Aufnahmeobjektes auf eine Detektoranordnung trifft, die mindestens einen röntgenbilderfassenden Detektor aufweist.

In der zahnärztlichen Röntgentechnik werden Verfahren und Vorrichtungen angewendet, mit denen es möglich ist, Schichtaufnahmen von einem Menschen, insbesondere vom Bereich des Kiefers, zu erstellen. Eine besondere Anwendung liegt in der Erstellung von Schichtaufnahmen, deren Schichtverlauf senkrecht zum Kieferbogen verläuft. Solche Schichtaufnahmen werden auch Transversalschnitte genannt. Im Vergleich zu den sonst üblichen Panorama-Schichtaufnahmen weisen solche Transversalschnitte einen besonders kleinen Tiefenschärfenbereich auf. In der EP-0 229 971 wird eine Vorrichtung, mit der solche Schichtaufnahmen auf einem Röntgenfilm möglich sind, aufgezeigt.

In der EP-0 632 994 wird eine Vorrichtung zur Erstellung von Röngenaufnahmen auf digitalem Weg beschrieben. Hierzu ist eine Zeilendetektorkamera mit einem Detektor vorgesehen, der als ein- oder mehrteiliger CCD-Sensor ausgeführt ist. Die Abmessungen der Detektoranordnung betragen, unabhängig davon, ob ein ein- oder mehrteiliger Sensor verwendet wird, typischerweise 135 bis 180 mm in der Bildhöhe und ca. 6 mm in der Bildbreite. Diese Maße berücksichtigen einerseits die für ei-ne gute Diagnose notwendige Bilderfassungsgröße, andererseits eine ausreichende Tiefenschärfe bei Betrachtung der einzelnen Schichten. In der Praxis hat sich gezeigt, daß das Blenden- und Detektorsystem so abgestimmt sein muß, daß der nutzbare Strahlenfächer für transversale Schichten, also für die oben-genannten Transversalschnitte, eine Breite von mindestens 20 mm in Detektorebene aufweisen muß, um einen Tiefenschärfenbereich von ca. 1 bis 3 mm erzielen zu können. Eine Detektoranordnung in der vorgenannten Größenordnung ist mit der heutigen Technologie vergleichsweise teuer.

Aus der US 4,188,537 ist eine dentale Röntgeneinrichtung bekannt, bei der ein sich in vertikaler Richtung erstreckender Zeilensensor oder ein einzelner Sensor zur Erstellung einer Röntgenaufnahme in vertikaler Richtung entlang einer Linie bewegt wird. Der Sensor kann dabei auch einer Drehbewegung folgen, so dass zusammen mit der vertikalen Bewegung der gesamte Bereich des Patienten durch Röntgenstrahlen erfaßt wird.

Ziel der vorliegenden Erfindung ist es, eine Vorrichtung zu schaffen, mit der es möglich ist, die eingangs erwähnten Schichtaufnahmen mit einer kleineren, kostengünstigeren Detektorfläche erstellen zu können.

Die Aufgabe wird mit den Mitteln des Anspruch 1 gelöst. Dabei sind mehrere Detektoren vorgesehen und diese sind beabstandet angeordnet. Eine solche Anordnung mit mehreren Detektoren ist immer noch kostengünstiger als eine Detektoranordnung in der eingangs genannten Bilderfassungsgröße.

Die Aufnahmeprozedur erfolgt in mehreren, zeitlich getrennten Abschnitten, in dem die aus mehreren Detektoren bestehende Detektoranordnung in verschiedenen Positionen angeordnet wird. Dadurch kann ein sehr viel kleinerer und damit kostengünstigerer Detektor eingesetzt werden.

Besonders wirtschaftlich ist es, wenn der verwendete Detektor die Abmessung des Detektors eines sog. Intraoral-Sensors aufweist, wie er heute für Intraoral-Aufnahmen verwendet wird oder wenn ein kompletter solcher Intraoral-Sensor dafür eingesetzt wird. Derartige Sensoren haben typischerweise Abmessungen von etwa 30 x 20 mm. Mit der zuletzt genannten Variante läßt sich der an sich für Intraoral-Aufnahmen bereits eingesetzte Sensor auch für extraorale Aufnahmen verwenden (dual use).

Wenn mehrere Transversal-Schichtaufnahmen erstellt werden sollen, kann es vorteilhaft sein, die Teilaufnahmen nicht nur in der Vorlaufphase sondern auch in der Rücklaufphase der die Röntgenstrahlenquelle und die Detektoranordnung tragenden Dreheinheit durchzuführen. Der gesamte Aufnahmeablauf läßt sich so wesentlich beschleunigen. Unter diesem Aspekt kann es auch vorteilhaft sein, eine Detektorverschiebung erst dann vorzunehmen, wenn alle Transversal-Schichtaufnahmen für eine Position, also für eine Teilaufnahmeposition der Detektoranordnung durchgeführt worden sind.

Anhand der Zeichnung werden mehrere Ausführungsbeispiele der Erfindung näher beschrieben. Es zeigen:
- Figur 1: ein zahnärztliches Röntgendiagnostikgerät mit der erfindungsgemäßen Vorrichtung in einer Seitenansicht,
- Figur 2: eine Prinzipskizze zur Erläuterung der mechanischen Zusammenhänge der Vorrichtung,
- Figuren 3, 4 und 5: Versionen einer Detektoranordnung, jedoch ohne die erfindungsgemäße zusätzliche strahler enfernte Detektoren
- Figur 6: eine Prinzipskizze zur Erläuterung von Transversalschnitten,
- Figur 7: ein Blockschaltbild,
- Figuren 8 und 9: die erfindungsgemäße Detektoranordnung.

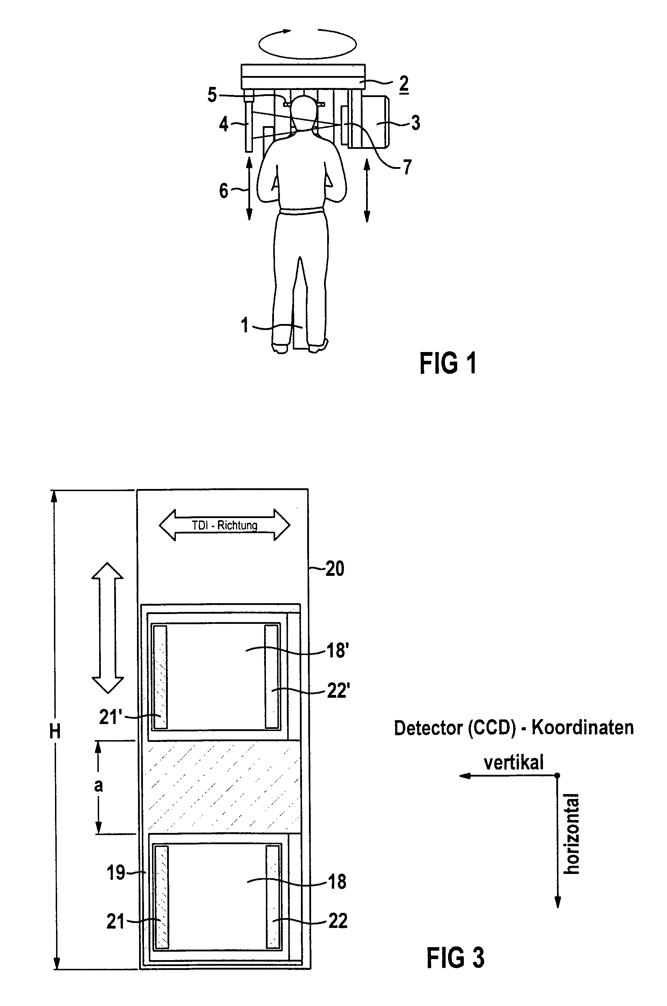

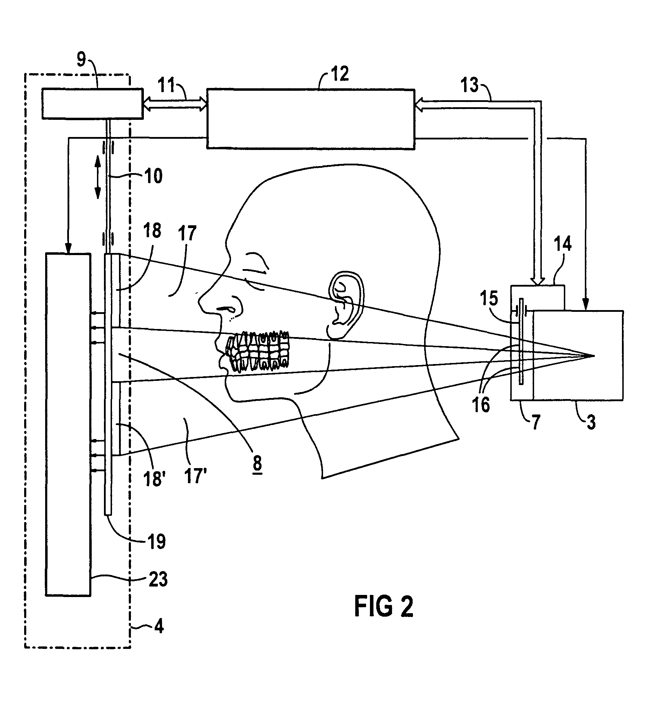

Die Figur 1 zeigt in einer Prinzipdarstellung ein zahnärztliches Röntgendiagnostikgerät zur Erstellung von Panorama-Schichtaufnahmen, welches gemäß der Erfindung auch zur Darstellung von Transversalschnitten eingesetzt werden kann. Das Gerät enthält eine in der Höhe verstellbare Tragsäule 1, an der eine Dreheinheit 2 gehaltert ist, die Träger einerseits einer Röntgenstrahlenquelle 3 und andererseits diametral dazu einer Röntgen-Detektorkamera 4 ist. Mit 5 ist eine Kopfhalte- und Positioniereinrichtung bezeichnet, mit der in bekannter Weise der Patientenkopf in einer definierten Position fixiert werden kann. Aufbau sowie Verstellmöglichkeiten der Dreheinheit und der Kopfhalte- und Positioniereinrichtung sind bekannt und beispielsweise in der eingangs genannten EP-0 632 994 beschrieben. Die Zeilenkamera 4 besteht aus einem länglichen Gehäuse mit einem nicht näher bezeichneten Schlitz an der der Strahlenquelle zugewandten Seite. Hinter dem Schlitz befindet sich im Innern der Kamera eine Detektoranordnung 8 mit einem oder mehreren strahlenempfindlichen Detektoren, z.B. in Form von CCD-Sensoren. Aufbau und Anordnung werden später noch näher erläutert. Die Detektoranordnung 8 ist innerhalb der Zeilendetektorkamera in Richtung ihrer Längsachse in Richtung des Pfeiles 6 verstellbar gehaltert. Im folgenden wird von einer Detektoranordnung mit zwei aktiven Detektorelementen ausgegangen. Synchron dazu ist auch ein mit 7 bezeichnetes Blendensystem, welches die Primärblende beinhaltet, verstellbar gehaltert. Die elektromechanische Verbindung der Zeilendetektorkamera 4 mit dem Blendensystem 7 wird anhand der Figur 2 näher erläutert.

Die im Innern angeordneten, später noch näher erläuterten Detektorelemente können mittels einer geeigneten Verstelleinrichtung, hier mittels eines Schrittmotors 9 und einer Spindel 10, entlang der Detektorhauptachse verstellt werden. Der Schrittmotor 9 kommuniziert über eine (serielle) Schnittstelle 11 mit einer Steuerelektronik 12 der Gerätesteuerung des Röntgengerätes. Die Steuerelektronik 12 gibt über eine weitere Schnittstelle 13 Steuerbefehle an einen an der Röntgenstrahlenquelle 3 angeordneten Stellantrieb 14. Mit diesem Stellantrieb erfolgt die synchrone Verstellung einer Primärblende 15 des Blendensystems 7. Die Primärblende 15 enthält zwei beabstandet angeordnete Blendenöffnungen 16. Die von der Strahlenquelle 3 erzeugten Röntgenstrahlen werden so in zwei Strahlenbündel 17, 17' unterteilt, die so fokussiert sind, daß sie exakt auf zwei im Innern der Zeilendetektorkamera 4 angeordnete Detektoren 18, 18' treffen. Die beiden Detektoren 18, 18' sind auf einem Träger 19 angeordnet, der, wie beschrieben, mittels der Verstelleinrichtung 9, 10 in der angegebenen Pfeilrichtung verstellbar ist.

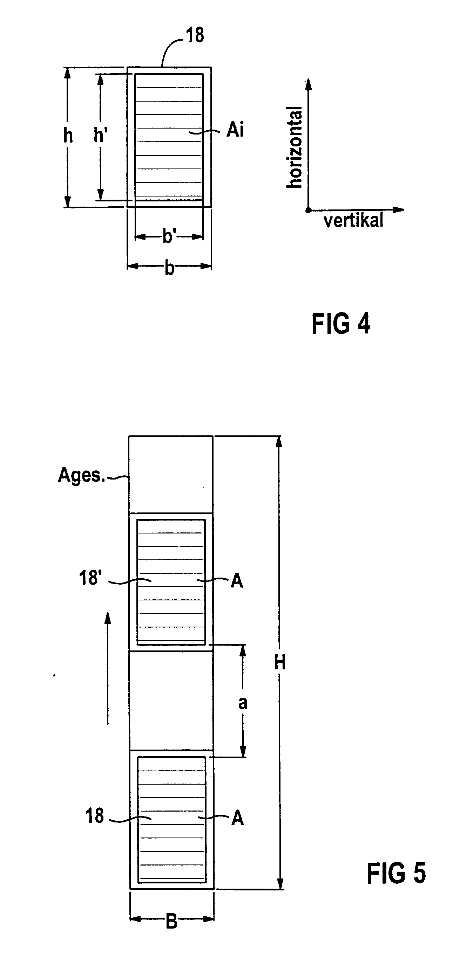

Die Figur 3 zeigt die Detektoranordnung in einer Frontansicht. Der Träger 19, auf dem die beiden Detektoren 18, 18' 9 befestigt sind, ist in einem Rahmen 20 verschiebbar gehaltert. Mit 21 bzw. 21' sind die Austaktregister der Detektoren bezeichnet, die, wie aus der Koordinatenbezeichnung rechts im Bild ersichtlich, als Horizontalregister ausgebildet sind, d.h. die TDI-Richtung (TDI steht für Time Delay and Integration und ist eine CCD-spezifische Technologie) verläuft quer zur Verschieberichtung. Mit 22, 22' sind zweite Ausleseregister bezeichnet, die gemäß einer vorteilhaften Variante dann vorgesehen werden müssen, wenn man das Austakten in der entgegengesetzten Richtung ausführen will, wenn man also Teilaufnahmen während der Rücklaufphase der Dreheinheit machen will.

Mit 23 in Figur 2 ist die Ansteuerelektronik für die beiden Detektoren 18, 18' bezeichnet.

Figur 4 zeigt einen einzelnen Detektor 18 mit den bei Intraoral-Sensoren üblichen Abmessungen. Solche Sensoren haben typischerweise eine Höhe (h) von 30 mm und eine Breite (b) von 20 mm. Die von Strahlung ausgeleuchtete Sensorfläche ist mit Ai bezeichnet und beträgt in der Höhe (h') 26 mm und in der Breite (b') 18 mm.

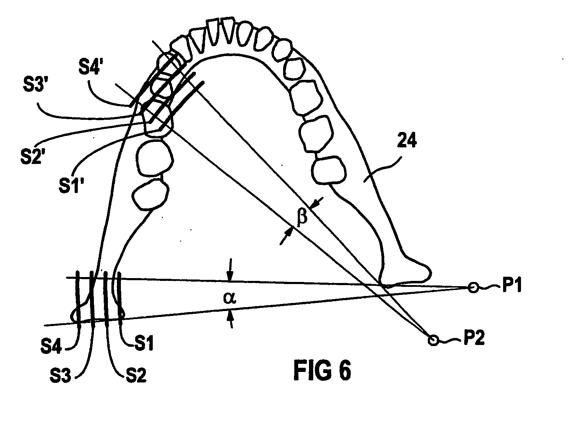

Die Figur 5 zeigt eine Detektoranordnung mit mehreren Detektoren. Die zur Objektaufnahme erforderliche Detektorfläche (Ages.) ist in n Flächen unterteilt, wobei die Teilfläche A dem n-ten Teil der gesamten Detektorfläche (Ages.) entspricht. Jede Teilfläche setzt sich aus m-Sensorflächen (Ai) zusammen, wobei m die Anzahl der verwendeten Detektoren repräsentiert. Im Anwendungsbeispiel sind zwei Detektoren 18, 18' vorgesehen. Die aus dieser Anordnung resultierende ausgeleuchtete Teilfläche A beträgt 2 x Ai. In Relation dazu ist die gesamte, für die Aufnahme eines Objekts erforderliche Detektorfläche mit Ages. bezeichnet. Es sei hier angenommen, daß diese Gesamtfläche (Ages.) H x B = 100 mm x 20 mm betragen soll. Die beiden Detektoren 18, 18' sind im Abstand (a) voneinander am Träger 19 befestigt, wobei der Abstand a so bemessen ist, daß sich die ausgeleuchteten Teilflächen zu einem kompletten Bild ergänzen, ohne daß eine Beeinträchtigung an den Bildübergangsstellen erkennbar ist. In der beispielsweise vorgeschlagenen Anordnung werden Detektor und Strahlenfächer jeweils um ca. 25 mm in Pfeilrichtung, d.h. entlang der Längsachse der Detektoren verschoben. Um die angegebene Gesamthöhe von etwa 100 mm zu erzielen, sind demnach zwei Aufnahmephasen notwendig, eine erste in der gezeigten Grundstellung und eine zweite, bei der die beiden Detektoren um ca. 25 mm in Pfeilrichtung verschoben sind.

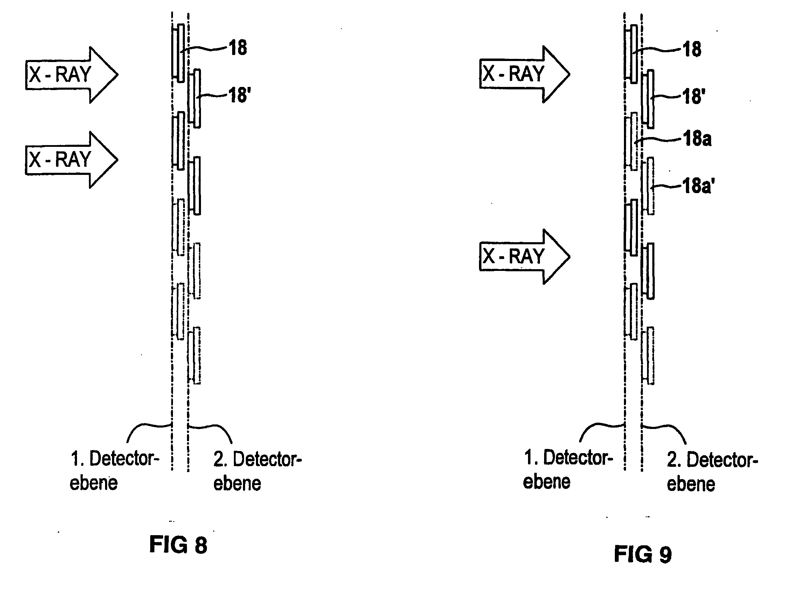

Um die Anzahl der Aufnahmephasen zu reduzieren, ist es vorgesehen, die Lücken, welche durch die mit Abstand a beabstandeten Detektoren entstanden sind, durch zusätzliche Detektoren teilweise oder vollständig zu schließen. Es wird dabei davon ausgegangen, daß ein bloßes Aneinanderfügen der Detektoren in der vorgegebenen Bilddetektorebene nicht ohne bildqualitätseinschränkende Einflüsse realisierbar ist, da an den Stoßstellen inaktive Strukturen entstehen, die nicht zur Bilderfassung beitragen. Um diese Nachteile zu vermeiden, wird vorgeschlagen, die Lücke durch Detektoren zu schließen und diese in einer zweiten, strahlerentfernteren Bilddetek-torebene anzuordnen. Die Figuren 8 und 9 zeigen eine solche Anordnung. Die Detektoren sind hier überlappend angeordnet, so daß ein größerer Teil oder die gesamte Zeile in einer Auf-nahmephase ausgeleuchtet werden kann.

Der Vorteil dieser Anordnung ist eine Reduzierung der Aufnahmephasenanzahl und der Verzicht auf eine aufwendige Blendensteuerung, da keine Teilbereiche ausgeblendet werden müssen. Da die Kosten pro aktive Detektorfläche überproportional mit denen der Detektorfläche ansteigen, kann der Einsatz weiterer, kleiner Detektoren eine wirtschaftliche Lösung sein.

Eventuell durch in der ersten Ebene angeordnete Detektoren hervorgerufene Verschattungen können, sofern diese durch inaktive Detektorstrukturen hervorgerufen werden, mit üblichen Mitteln der Bildverarbeitung, wie z.B. der Gain- und Blemish-Korrektur, korrigiert werden.

Neben anderen Parametern ist durch die Lage der Bilddetektorebenen die aufzulösende Schicht definiert. Werden, wie beschrieben, zwei oder sogar noch weitere, unterschiedliche Detektorebenen zur Erfassung einer Schicht benutzt, so kann dies negativ die gewünschte Schichtauflösung beeinflussen. In der Praxis hat es sich gezeigt, daß ein Versatz von bis zu 3 mm keine kompensierenden Maßnahmen erforderlicht macht. Zur Kompensation können die Ladungen mit unterschiedlichen Geschwindigkeiten im Detektor bewegt werden. Im Falle der TDI-Technologie werden die Ladungen in den Detektoren der zweiten Ebene mit einer höheren TDI-Taktrate schneller bewegt, als die Ladung in jenen Detektoren, die näher zum Strahler angeordnet sind. Analog dazu kann diese Kompensation auch im "full frame mode" erfolgen.

In diesem Falle sind vier Aufnahmephasen mit viermaliger Verschiebung des Detektors um jeweils ca. 25 mm erforderlich.

Anhand der Figur 6 wird der Ablauf zur Erzielung von Transversalschichtaufnahmen näher erläutert. Es wird dabei davon ausgegangen, daß vier Transversalschnitte in den vier angegebenen Schichtebenen 1, 2, 3 und 4 aufgenommen werden sollen.

Die Bedienperson wählt zunächst die Aufnahmeparameter (unter-suchter Bereich, Anzahl, Qualität und Größe der Transversalschnitte sowie Dosis). Diese Parameter können am Röntgengerät oder an einem angeschlossenen PC durch Auswahl vordefinierter Programme oder auch durch manuelles Zusammenstellen der Parameter erfolgen. Nach Positionierung des Patienten in der Kopfhalte- und Positoniereinrichtung 5 (Fig. 1) wird der Aufnahmeablauf gestartet. Das Gerät justiert sich zunächst durch Anfahren von Referenzpunkten und positioniert sich anschließend in einer Startposition für die nachfolgende Aufnahmeserie. Die Anzahl der Einzelaufnahmen ergibt sich durch die Anzahl der Transversal-Schichtaufnahmen und die Anzahl der Aufnahmephasen. Im Ausführungsbeispiel nach Figur 6 sei angenommen, daß von dem aufgezeichneten Kieferbogen 23 zunächst vier verschiedene Schichtaufnahmen S1 bis S4 vom linken Kiefergelenk und danach vier weitere Schichtaufnahmen S1' bis S4' von einem weiteren Kieferabschnitt erstellt werden sollen. Die Dreheinheit 2 wird zunächst in die Position P1 gefahren, von der aus von der Strahlenquelle Strahlen auf den gewünschten Abbildungsbereich abgegeben werden können, der beispielsweise den eingezeichneten Winkel a umfassen soll. Die Schichtaufnahme S1 wird erstellt, indem beginnend von einer Ausgangsposition über den Winkel a gestrahlt wird. Nachdem die Aufnahme für die Schichtlage S1 erstellt ist, wird die Strahlenquelle abgeschaltet und die Dreheinheit um den Schwenkwinkel a wieder zurück in die Ausgangsposition gefahren. Danach werden in der Folge die Schichtlagen S2, S3 und S4 erstellt. Andere Schichtlagen für weitere Objektausschnitte können analog erstellt werden, beispielsweise wie dargestellt, von der Position P2 aus. Der kinematische Bewegungsablauf der die Strahlenquelle und Zeilendetektorkamera aufnehmenden Dreheinheit ist an sich bekannt, ebenso die dazu erforderliche Ansteuerung der Detektoren nach dem TDI-Verfahren, um die gewünschte Schichtung zu erhalten.

Bei der zuletzt beschriebenen Aufnahmeprozedur wird die eigentliche Aufnahme mit Strahlung während des Vorlaufs der Dreheinheit durchgeführt. In der Rücklaufphase ist die Strahlung abgeschaltet oder sie wird durch eine Blendenverstellung unwirksam gemacht.

Um die vier Schichten im Ausführungsbeispiel erfassen zu können, gibt es mehrere Möglichkeiten. Die eine besteht darin, zunächst eine Schicht komplett zu erstellen, d.h. bei einer Detektoranordnung mit zwei Detektoren zunächst zwei Aufnahmesequenzen mit Detektorverschiebung durchzuführen und entsprechend danach die weiteren Schichten S2, S3 und S4 aufzunehmen.

Alternativ dazu besteht die Möglichkeit, in einer Detektorposition alle vier Schichten aufzunehmen, danach die Detektoranordnung zu verschieben und anschließend wiederum alle vier Schichten in der zweiten Detektoranordnung aufzunehmen. Diese Version hat den Vorteil, daß weniger Bewegungsvorgänge notwendig sind, die unter Umständen den gesamten Ablauf beschleunigen.

Wie eingangs bereits angesprochen, kann es vorteilhaft sein, während des Rücklaufes der Dreheinheit Schichtaufnahmen durchzuführen. Dazu ist es notwendig, die Detektoren mit einem zweiten Ausleseregister (Pos. 22, 22' in Fig. 3) auszustatten, um den TDI-Prozeß auch in der entgegengesetzten Richtung ausführen zu können.

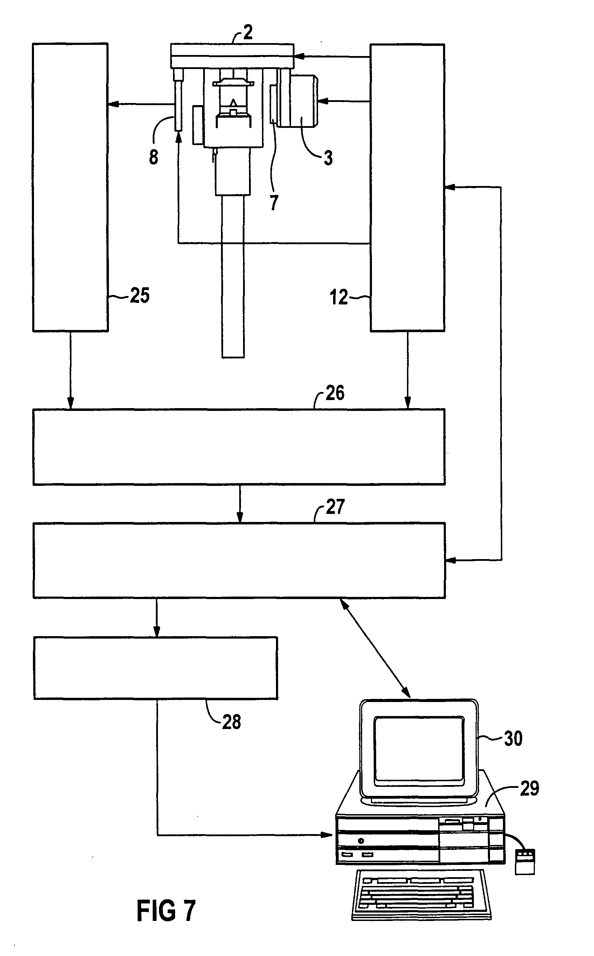

Die Figur 7 zeigt ein Blockschaltbild der erfindungsgemäßen Vorrichtung und verdeutlicht die Beziehung zwischen den einzelnen Komponenten. Die Gerätesteuerung 12 erzeugt Steuersignale für die Detektoranordnung 8, das Blendensystem 7 und Röntgenquelle 3 und koordiniert für jede Teilbildsequenz den Ablauf bzw. die Bewegung der Detektoranordnung 8 und des Strahlers 3 entsprechend der vorbestimmten Bahn bei einer Aufnahme. Die während der Sequenz erfaßten Bilddaten werden in einen Zwischenspeicher 25 abgelegt. Wie durch die Pfeilangaben erkennbar, steuert die Gerätesteuerungselektronik 12 nicht nur den Strahler 3, das Blendensystem 7 und die Detektoranordnung 8, sondern liefert gleichsam aufnahmebezogene Strahlungs-, Positions- und Bahndaten an eine Software 26. Diese verarbeitet auch die in dem Zwischenspeicher 25 abgelegten Bilddaten der Teilbilder, die mit den Detektoren gewonnen worden sind, zu einem 'Rohbild'. Dieses 'Rohbild' wird anschließend in einer weiteren Software 27 zu einem Röntgenbild verarbeitet, welches danach in einer Datenbank 28 abgelegt wird. Von dort aus kann es beispielsweise über einen PC 29 abgerufen und auf einem Monitor 30 dargestellt werden. Vom PC 29 können vorteilhafterweise durch die für eine Aufnahme notwendigen Parameter aus der Software 27 generiert werden, die von dort aus an die Gerätesteuerung weitergeleitet werden.

Die zur Erzeugung der Schichtbilder notwendige Summation bzw. Integration kann - wie aufgezeigt - mit Hilfe der CCD, die im sog. TDI-Modus beschrieben werden, erfolgen. Hierbei entsteht das Bild während der Datenerfassung durch eine 'a-naioge' Summation innerhalb der aktiven CCD-Fläche.

Die Bilderfassung ist alternativ auch ohne TDI-Verfahren auch möglich, indem für jedes Bewegungsinkrement entsprechend der notwendigen Sensorbewegung ein Abbild der kompletten Sensorfläche erfaßt und gespeichert wird. Für diesen Betriebsmodus wird üblicherweise der Begriff 'full frame mode' oder 'area mode' verwendet. Statt CCD-Technologie kann bei dieser Alternative auch auf eine kostengünstigere Technologie (z.B. CMOS-Technik) zurückgegriffen werden. Die zur Entstehung einer scharfen Schicht notwendige Summation von Bildpunkten erfolgt in diesem Falle nach der Datenerfassung in einer entsprechend ausgelegten Recheneinheit.

Die bei jeder Aufnahme erzeugten Signale eines Teilbildes werden nach Zwischenspeicherung von einer Signalverarbeitungssoftware zu einem Schichtbild verarbeitet. Jede Transversalaufnahme wird also aus den zugehörigen Aufnahmen von einem Rechner zusammengesetzt, bearbeitet und abgespeichert und sodann auf einem Bildschirm dargestellt. Beim Zusammensetzen sind dem Rechner die geometrischen Positionen während der Aufnahmesequenzen und auch die Abmessungen des Detektors bekannt.

Wie eingangs erwähnt, sollte die Detektorbreite mind. 20 mm aufweisen, um Mindest-Anforderungen an die Tiefenschärfe der aufzulösenden Transversalschicht zu erfüllen. Durch Querverschieben der vorgenannten Detektorstrukturen und durch Wiederholung der beschriebenen Aufnahmeprozeduren kann die wirksame Detektorbreite vergrößert und somit die Tiefenschärfe der Transversalschicht verbessert werden.

Mit jeder Querverschiebung entsteht ein weiterer Aufnahmesatz. Diese Aufnahmesätze können, wie beschrieben, mit dem "analogen" TDI-Verfahren oder "digitale" Summation erstellt werden. Um die Querverschiebung mit zu berücksichtigen, müssen diese Aufnahmesätze ebenfalls aufsummiert werden. Zur Summation der digitalisierten Aufnahmesätze ist verständlicherweise nur das "digitale" Verfahren sinnvoll anwendbar.

Wie bereits erwähnt, erfolgt die Primärblendeneinstellung synchron zu der Verstellung der Detektoren. Dies kann durch eine motorische verschiebbare Schablone in der in Figur 2 dargestellten Weise erfolgen. Alternativ kann auch die Blende durch eine entsprechende Verstellung einer unteren und oberen Abgrenzung erfolgen; ebenso ist es denkbar, eine drehbare Blende mit an unterschiedlichen Stellen befindlichen Blendenöffnungen vorzusehen.

## Patentansprüche

1. Einrichtung zur Erstellung von Röntgenaufnahmen von Körperteilen eines Menschen, insbesondere von Röntgen-Schichtaufnahmen vom Kiefer oder Schädel eines Patienten,
- mit einer Strahlenquelle (3) zum Erzeugen eines Strahlbündels, der gegenüberliegend eine Detektoranordnung (8) mit mindestens einem Detektor (18,18') angeordnet ist,
- mit einer Primärblende (15) mit einer Blendenöffnung (16,16') zum Begrenzen des Strahlenbündels,
- mit einer Verstelleinrichtung, die die Detektoranordnung (8) entlang der Längsachse und/oder Querachse der Detektorfläche verschieben kann und dabei synchron die Blendenöffnung (16,16') der Primärblende verschieben kann,
**dadurch gekennzeichnet, daß** mehrere in einem Abstand (a) voneinander beabstandet angeordnete Detektoren (18, 18a) vorgesehen sind, wobei zusätzliche Detektoren (18', 18a') vorgesehen sind, die strahlerentfernt in den Lücken zwischen den um den Abstand (a) beabstandeten, strahlernäheren Detektoren (18, 18a) plaziert sind, so dass die Lücken mit den strahlerentfernten Detektoren (18', 18a') teilweise oder vollständig geschlossen sind.

2. Einrichtung nach Anspruch 1, bei der der Abstand (a) kleiner oder gleich dem Maß der ausgeleuchteten Detektorhöhe (h') ist.

3. Einrichtung nach Anspruch 1 oder 2, bei der eine Detektoranordnung (8) mit mindestens einem Detektor in den Abmessungen eines Intraoralsensors (31) verwendet ist.

4. Einrichtung nach einem der Ansprüche 1 bis 3, bei der zwei weitere, strahlerfernere Detektoren vorgesehen sind, die in Verlängerung der Längsachse jeweils an den Enden der zeilenförmigen Detektoranordnung (8) zusätzlich zu den strahlernäheren Detektoren (18, 18a) überlappend angeordnet sind.

5. Einrichtung nach einem der Ansprüche 1 bis 4, bei der die Detektoren (18, 18', 18a, 18a') aus überlappenden Detektorstrukturen bestehen.

6. Einrichtung nach einem der Ansprüche 1 bis 5, bei der die Summation bzw. Integration der Teilaufnahmen entsprechend der Detektorebene und der gewünschten Schichtauflösung erfolgt.

7. Einrichtung nach einem der Ansprüche 1 bis 6, bei der der verwendete Detektor (18; 31) mit einem zweiten Ausleseregister (22, 22') ausgestattet ist und in seiner Integrationsrichtung umschaltbar ausgebildet ist.

## Claims

1. Device for producing X-ray images of parts of the human body, in particular tomographic X-ray images of a patient's jaw or skull,
- having a radiation source (3) for generating a beam which is arranged opposite a detector arrangement (8) with at least one detector (18, 18'),
- having a primary diaphragm (15) with a diaphragm aperture (16, 16') for limiting the beam, and
- having an adjusting device which can displace the detector arrangement (8) along the longitudinal axis and/or transverse axis of the detector surface, and in doing so can synchronously displace the diaphragm aperture (16, 16') of the primary diaphragm,
**characterized in that** a plurality of detectors (18, 18a) are arranged separated from one another at a spacing (a), additional detectors (18', 18a') being provided which are placed remote from the radiator in the gaps between the detectors (18, 18a) near the radiator and separated by the spacing (a), such that the gaps are partially or completely closed by the detectors (18', 18a') remote from the radiator.

2. Device according to Claim 1, in which the spacing (a) is smaller than or equal to the dimension of the illuminated detector height (h').

3. Device according to Claim 1 or 2, in which a detector arrangement (8) with at least one detector is used in taking measurements with an intraoral sensor (31).

4. Device according to one of Claims 1 to 3, in which two further detectors remote from the radiator are provided which are arranged overlapping on an extension of the longitudinal axis, in each case at the ends of the rectilinear detector arrangement (8) in addition to the detectors (18, 18a) near the radiator.

5. Device according to one of Claims 1 to 4, in which the detectors (18, 18', 18a, 18a') comprise overlapping detector structures.

6. Device according to one of Claims 1 to 5, in which the summation or integration of the partial images is performed in accordance with the detector plane and the desired layer resolution.

7. Device according to one of Claims 1 to 6, in which the detector (18; 31) used is fitted with a second read out register (22, 22') and is designed such that its integration direction can be switched over.

## Revendications

1. Installation pour produire des radiographies de parties du corps humain, en particulier des tomographies de la mâchoire ou du crâne d'un patient,
- ayant une source de rayonnement (3) qui est destinée à produire un faisceau de rayons et qui est placée en face d'un dispositif de détection (8) ayant au moins un détecteur (18, 18'),
- ayant un diaphragme primaire (15) ayant une ouverture de diaphragme (16, 16') pour délimiter le faisceau de rayons,
- ayant une installation de déplacement qui peut déplacer le dispositif de détection (8) le long de l'axe longitudinal et/ou de l'axe transversal de la surface de détecteur et qui peut de ce fait déplacer de façon synchrone l'ouverture (16, 16') du diaphragme primaire
**caractérisé en ce que** l'on prévoit plusieurs détecteurs (18, 18a) disposés selon un intervalle (a) entre eux, en prévoyant des détecteurs supplémentaires (18', 18'a) qui sont placés, à distance des émetteurs, dans les espaces entre les détecteurs (18, 18a) proches des émetteurs et disposés selon un intervalle (a), de sorte que les espaces entre les détecteurs (18', 18'a) placés à distance des émetteurs soient partiellement ou complètement comblés.

2. Installation selon la revendication 1, dans laquelle l'intervalle (a) est inférieur ou égal à la dimension de la hauteur éclairée de détecteurs (h').

3. Installation selon la revendication 1 ou 2, dans laquelle on utilise un dispositif de détection (8) ayant au moins un détecteur dans les dimensions d'un capteur intraoral (31).

4. Installation selon l'une des revendications 1 à 3, dans laquelle il est prévu, en plus des détecteurs (18, 18a) proches des émetteurs, deux autres détecteurs éloignés des émetteurs, qui sont placés en chevauchement dans le prolongement de l'axe longitudinal à chacune des extrémités du dispositif de détection (8) disposé en ligne.

5. Installation selon l'une des revendications 1 à 4, dans laquelle les détecteurs (18, 18', 18a, 18a') sont composés de structures de détection en chevauchement.

6. Installation selon l'une des revendications 1 à 5, dans laquelle l'addition ou l'intégration des radiographies partielles est réalisée en fonction du plan de détection et de la résolution souhaitée de la couche.

7. Installation selon l'une des revendications 1 à 6, dans laquelle le détecteur (18 ; 31) utilisé est muni d'un registre de lecture (22, 22') et est configuré de façon réversible dans sa direction d'intégration.
